Europäisches Patentamt

European Patent Office

Office européen des brevets

(19)

(11) EP 0 956 509 B1

(12) FASCICULE DE BREVET EUROPEEN

(45) Date de publication et mention
de la délivrance du brevet:
**26.09.2001 Bulletin 2001/39**

(21) Numéro de dépôt: **98929499.6**

(22) Date de dépôt: **05.06.1998**

(51) Int Cl.⁷: **G01N 33/86**

(86) Numéro de dépôt international:
**PCT/FR98/01148**

(87) Numéro de publication internationale:
**WO 98/55875 (10.12.1998 Gazette 1998/49)**

(54) **UTILISATION DE VENIN DE CROTALUS VIRIDIS HELLERI, PROCEDE ET NECESSAIRE POUR LA DETERMINATION DE LA REACTIVITE DE LA PROTEINE C ACTIVEE**

VERWENDUNG VON CROTALUS VIRIDIS HELLERI VENOM ZUR BESTIMMUNG VON AKTIVIERTEM PROTEIN C REAKTIVITÄT

USE OF CROTALUS VIRIDIS HELLERI VENOM, METHOD AND KIT FOR DETERMINING THE REACTIVITY OF ACTIVATED PROTEIN C

(84) Etats contractants désignés:
**DE ES FR IT SE**

(30) Priorité: **06.06.1997 FR 9707041**

(43) Date de publication de la demande:
**17.11.1999 Bulletin 1999/46**

(73) Titulaire: **SOCIETE DIAGNOSTICA-STAGO F-92600 Asnières (FR)**

(72) Inventeur: **VAN DREDEN, Patrick F-94700 Maisons-Alfort (FR)**

(74) Mandataire: **Clisci, Serge et al S.A. FEDIT-LORIOT & AUTRES CONSEILS EN PROPRIETE INDUSTRIELLE 38, Avenue Hoche 75008 Paris (FR)**

(56) Documents cités:
WO-A-93/10261    WO-A-96/04560
WO-A-96/15457    US-A- 5 318 899
US-A- 5 342 830

• DATABASE WPI Section Ch, Week 9214 Derwent Publications Ltd., London, GB; Class B04, AN 92-112275 XP002057015 & SU 1 659 486 A (MOSCOW LOMONOSOV UNIV) , 30 juin 1991
• DATABASE WPI Section Ch, Week 9241 Derwent Publications Ltd., London, GB; Class B04, AN 92-334415 XP002057016 & HU 60 393 A (VINCZE Z), 28 août 1992

**Description**

*Domaine de l'invention*

La présente invention a trait à une nouvelle utilisation d'un venin de serpent particulier, le venin de **Crotalus viridis helleri** (ce venin est désigné ci-après CVH), dans le domaine de la détermination de la réactivité de la protéine C activée, cette détermination englobant celle de l'activité de la protéine C fonctionnelle et celle de la résistance à la protéine C activée (la protéine C activée est notée ici APC et la résistance à APC est notée APC-R : les autres abréviations utilisées sont définies plus loin). L'invention concerne également un procédé de détermination de APC-R utilisant CVH et un nécessaire de dosage permettant de mettre en oeuvre ce procédé.

*Art antérieur*

L'étude des thrombophilies héréditaires a permis de démontrer que APC-R est principalement associée à une mutation sur le gène codant pour le facteur V. Cette mutation, dénommée "mutation de Leiden", "mutation R506Q", ou "mutation $^{506}R \rightarrow ^{506}Q$", se traduit au niveau de la séquence des aminoacides du facteur V humain par le remplacement en position 506 de Arg par Gln, et favorise l'apparition de thromboses le plus souvent veineuses (au niveau des membres inférieurs avec, le cas échéant, embolie pulmonaire). Voir notamment à cet effet les publications de R. M. BERTINA et al., *Nature*, *1994, 369, pages 64-68,* de M. KALAFATIS et al., *J. Biol. Chem*., *1994*, *269*, *pages 31869-31880, de* H. DE RONDE et al., *Thromb. Haemost*., *1994, 72*, *pages 880-886*, de P.J. SVENSSON et al., *N. Engl. J. Med*., *1994*, *330*, *pages 517-522,* de B, DAHLBÄCK et al., *Proc. Natl. Acad. Sci. USA*, *1993*, *90*, *pages 1004-1008* et de DAHLBÄCK et al., *Proc. Natl. Acad. Sci. USA*, *1994*, *91*, *pages 1396-1400.*
D'autres anomalies du facteur V peuvent être à l'origine de APC-R. Il se pourrait en particulier qu'un clivage à un moment inopportun en position 306 (i.e. $^{306}$Arg) de la séquence des aminoacides du facteur V et/ou Va soit l'une des autres causes possibles de APC-R.
La détermination de APC-R a été mise en oeuvre initialement (notamment selon un test APTT, KCCT ou analogue) pour apprécier l'existence d'un désordre thrombotique en comparant le temps de coagulation, qui est prolongé par addition de APC, d'un plasma témoin avec celui, qui est réduit en cas d'anomalie par rapport au précédent, d'un échantillon de plasma humain à tester additionné de APC.
Voir à cet effet : C.A. MITCHELL et al., *N. Engl. J. Med., 1987*, *317. pages 1638-1642*, L. AMER et al., *Thromb. Res*., *1990, 57*, *pages 247-258* et B. DAHLBÄCK et al., *Thromb. Haemost*., *1991, 65*, *abstract 39*, *page 658.*
Le seul nécessaire actuellement disponible sur le marché (il est commercialisé sous la dénomination de "COATEST APC-RESISTANCE" par la société dite CHROMOGENIX) met en oeuvre le procédé décrit dans la divulgation de B, DAHLBÄCK et al., *Thromb. Haemost*., *1991*, *65*, *abstract 39*, *page 658*, précitée et illustré dans WO-A-9310261 et l'article de B. DAHLBÄCK et al. *Proc. Natl. Acad. Sci. USA*, *1993*, *90*, *pages 1004-1008* précité. Ce procédé comprend le mélange d'un volume de plasma à tester ou de plasma témoin avec un volume de réactif APTT [PL + activateur de surface (silice, kaolin ou verre)], l'incubation pendant 4 minutes à 37°C, puis l'initiation de la coagulation au moyen d'un volume d'une solution de $CaCl_2$, d'une part, ou d'un volume d'une solution de $CaCl_2$ et APC, d'autre part.
Ce procédé est insatisfaisant en ce sens que, sensible notamment à la présence du facteur VIII, d'héparine, de PS, des CAC et des VKA, il fournit trop de résultats faux négatifs et faux positifs. Des perfectionnements ont donc été proposés dans WO-A-9615457, WO-A-9604560 et EP-A-0711838.
WO-A-9615457 propose un procédé de détermination de APC-R dans lequel on met en contact le plasma à tester, un réactif procoagulant [qui est ici le facteur tissulaire (le produit préféré) ou RVV] et un VdfP, puis après incubation on initie la coagulation au moyen de $CaCl_2$ ou $CaCl_2$ + APC, la variation du temps de coagulation étant ensuite appréciée par rapport à un plasma témoin.
WO-A-9604560 vise une technique améliorée pour détecter APC-R. Cette technique est fondée sur l'utilisation d'un réactif exogène activant spécifiquement le facteur V en Va, en complément d'une activation de la coagulation, soit par la transformation X→Xa, soit par la transformation prothrombine→thrombine selon un mécanisme dépendant du facteur V.
WO-A-9604560 préconise notamment

- en tant que réactif exogène, un venin de serpent tel que le venin de **Naja nivea** pour activer V en Va, et
- pour l'activation complémentaire : soit RVV-X pour activer X en Xa, soit un venin (ou extrait de venin) de serpent, tel que les venins de **Pseudonaja textilis**, **Notechis scutatus** ou **Oxyuranus scutellatus**, pour l'activation de la prothrombine en thrombine selon un mécanisme dépendant du facteur V.

EP-A-0711838 recommande pour la détermination de APC-R un procédé comprenant :

(a) le mélange d'un plasma à tester avec un réactif A à teneur réduite en facteur V, notamment VdfP,

(b) l'addition d'un réactif B activant directement ou indirectement le facteur V en Va, notamment un venin de serpent tel que RVV (qui par sa fraction RVV-V induit l'activation de V en Va et qui par sa fraction RVV-X induit l'activation de X en Xa) ou le venin de ***Echis carinatus*** (qui induit l'activation de la prothrombine en thrombine et par suite active de façon indirecte V en Va),

(c) l'addition d'un réactif C permettant la dégradation du facteur Va, à savoir APC [ou le mélange PC + activateur (notamment ACC)], et

(d) l'addition de réactifs permettant la détermination de l'activité résiduelle du facteur Va,

avec la condition que la proportion du volume de l'échantillon de plasma à tester par rapport au volume total soit au plus de 20 %, avantageusement inférieure ou égale à 10 % ("wobei der Anteil des Probenvolumens am gesamten Testvolumen maximal 20 %, vorzugsweise jedoch weniger oder gleich 10 % beträgt", selon la revendication 1, page 13 lignes 39-40 de EP-A-0711838).

Ce procédé présente l'inconvénient de faire appel à des volumes de plasma (à tester ou témoin), de réactif A et de réactif B qui sont tous les trois différents. Cette circonstance ne facilite pas les mesures des temps de coagulation au moyen de dispositifs totalement automatiques.

Par ailleurs, le document US-A-5342830 se propose de prévenir les thromboses induites par la fixation du facteur de von Willebrand (vWF) sur les plaquettes. Dans ce but, il préconise des venins de 40 serpents et mieux des peptides purifiés et isolés de ces venins, qui (i) inhibent la fixation de vWF sur des récepteurs plaquettaires mais (ii) n'inhibent pas la fixation du fibrinogène sur d'autres récepteurs plaquettaires.

Parmi ces venins, US-A-5342830 cite RVV et CVH, cependant il ne décrit ni ne suggère l'utilisation de CVH pour initier la coagulation par l'activation du facteur X en Xa.

Les procédés précités de WO-A-9604560 et EP-A-0711838 permettent de pallier à la majorité des inconvénients du test "COATEST APC-RESISTANCE". Cependant, ces deux documents ne décrivent ni ne suggèrent l'utilisation du venin de serpent particulier de l'invention, à savoir CVH, pour initier la coagulation au niveau du facteur X par la transformation X→Xa.

La seule technique efficace à ce jour pour déterminer APC-R est fondée sur la biologie moléculaire (MB). Elle est longue, fastidieuse, onéreuse et exige un matériel élaboré et un personnel hautement qualifié. En outre, sa mise en oeuvre doit satisfaire à l'article L. 145-15 de la loi française No. 94-653 du 29 juillet 1994 qui fait obligation d'obtenir le consentement écrit des personnes pour lesquelles une étude génétique est entreprise.

### But de l'invention

On se propose de fournir une nouvelle solution technique pour la détermination de APC-R, qui (i) soit simple sur le plan de la mise en oeuvre, (ii) soit au moins aussi efficace que celles envisagées dans les documents WO-A-9604560 et EP-A-0711838 précités, et (iii) s'appuie sur l'initiation de la coagulation au niveau du facteur X (i.e. transformation X→Xa) dont l'intérêt a été reconnu dans WO-A-9604560 (voir page 5 lignes 1-12).

Le but recherché est d'améliorer la sensibilité en réduisant le nombre de résultats faux positifs et faux négatifs. Dans cette optique, on se propose de mettre en oeuvre un procédé de détermination de APC-R selon des mécanismes classiques mais avec la particularité que l'on utilise un moyen ou réactif nouveau pour initier la coagulation par la transformation X→Xa, ce moyen ou réactif améliorant la sensibilité.

### Objet de l'invention

La nouvelle solution technique selon l'invention repose sur le choix d'un activateur particulier pour initier la coagulation au niveau du facteur X (i.e. X→Xa). Cet activateur particulier est un venin de serpent, à savoir CVH.

Selon un premier aspect de l'invention, l'on préconise une nouvelle utilisation d'un venin de serpent pour déclencher la coagulation par activation du facteur X en Xa en vue de déterminer la réactivité de la protéine C activée, notamment l'activité fonctionnelle de la protéine C (PC), d'un plasma humain à tester, ladite utilisation étant caractérisée en ce que l'on fait appel à du venin de ***Crotalus viridis helleri*** en tant que substance initiant la coagulation, en présence de $Ca^{2+}$, au niveau du facteur X, ledit venin activant sélectivement X en Xa sans être influencé par la présence ou l'absence des autres facteurs de la coagulation.

Selon un second aspect de l'invention, l'on préconise un nouveau procédé de détermination de APC-R d'un échantillon de plasma humain à tester, ledit procédé, qui met en oeuvre l'initiation de la coagulation au niveau du facteur X au moyen d'un venin de serpent en présence (i) de $Ca^{2+}$ ou (ii) d'un mélange de $Ca^{2+}$ et APC exogène, puis l'évaluation du temps de coagulation, (i) en l'absence de APC et, respectivement, (ii) en présence de APC, par rapport à un plasma normal, comprenant les étapes consistant à

(1°) mettre en contact

    (a) un échantillon de plasma humain à tester,
    (b) un réactif déficient en facteur V (VdfR) en tant que produit fournissant la majorité des (de préférence tous les) facteurs de la coagulation autres que le facteur V, et
    (c) CVH en tant que produit activant spécifiquement X en Xa, et incuber le mélange ainsi obtenu pendant au moins 1 minute à une température comprise entre 10 et 45°C, de préférence pendant 3 à 5 minutes à une température de 35 à 40°C, et mieux pendant 4 minutes à 37°C ;

(2°) introduire dans ledit mélange ainsi incubé (i) $Ca^{2+}$ ou, respectivement, (ii) $Ca^{2+}$ + APC exogène ;
(3°) évaluer le temps de coagulation (i) en l'absence de APC et, respectivement, (ii) en présence de APC ;
(4°) reproduire les étapes (1°) à (3°) en remplaçant à l'étape (1°) ledit échantillon de plasma humain à tester par un plasma normal en tant que témoin ; et,
(5°) apprécier APC-R par la comparaison des évaluations obtenues aux étapes (3°) et (4°).

Selon un troisième aspect de l'invention, l'on préconise une variante de ce procédé, qui est réalisable directement sans qu'il soit nécessaire, d'une part, d'établir une comparaison avec un plasma témoin (plasma normal ou pool de plasmas normaux) et, d'autre part, d'entreprendre de mesure en l'absence d'ajout de APC, Cette variante comprend les étapes (1°), (2°, ii) et (3°, ii) du procédé principal, d'une part, puis l'appréciation de APC-R par comparaison du temps de coagulation (T) obtenu à l'étape (3°, ii) à un temps de coagulation de référence (To) déterminé expérimentalement (et ayant notamment de façon statistique une valeur d'environ 110 s), d'autre part,
T < To indiquant généralement que l'échantillon de plasma humain à tester présente une résistance à la protéine C activée, et
T > To indiquant généralement que l'échantillon de plasma humain à tester ne présente aucune résistance à la protéine C activée.

Cette variante est avantageuse en ce sens que la détermination de APC-R est plus simple, moins coûteuse et plus facilement automatisable que le procédé principal sus-visé.

Selon un autre aspect de l'invention, l'on préconise un nécessaire, trousse ou kit de dosage caractérisé en ce qu'il comprend :

-     un premier réactif qui est un plasma déficient en facteur V,
-     un second réactif constitué par le CVH, et
-     un troisième réactif constitué par APC en milieu calcique.

### Abréviations

Par commodité on a utilisé dans la présente description les abréviations et acronymes suivants.

| | |
|---|---|
| ACC | venin d'**Agkistrodon contortrix**, ce venin est un produit qui active sélectivement PC en APC |
| APC | protéine C activée |
| APC-R | résistance à la protéine C activée (quand il est question de "plasma APC-R" on entend que ledit plasma est résistant à APC) |
| APTT | temps de tbromboplastine partielle activée (de l'anglais : "activated partial thromboplastin time") |
| CAC | anticoagulant circulant |
| CVH | venin de **Crotalus viridis helleri** |
| KCCT | temps de céphaline-kaolin (de l'anglais : "kaolin-cephalin clotting time") |
| MB | test par biologie moléculaire mettant en oeuvre ici une sonde ("probe") nucléique codant pour un fragment pepadique d'une dizaine d'aminoacides englobant $^{506}Q$, et la détection de la mutation par hybridation de la sonde après amplification (par PCR) de la région du DNA correspondant |
| MB+ | désigne un plasma dans lequel le facteur V comprend la mutation R506Q (i.e. plasma "positif" selon le test MB) |
| MB- | désigne un plasma dans lequel le facteur V ne comprend pas la mutation R506Q (i.e. plasma "négatif" selon le test MB) |
| OKB | tampon d'Owren-Koller, il s'agit d'un tampon véronal |
| PB | tampon Prionex (tampon contenant la fraction polypeptidique purifiée de collagène de porc) |
| PC | protéine C |
| PCR | amplification en chaîne par polymérase (de l'anglais : "polymerase chain reaction") |
| PL | phospholipides |
| PS | protéine S |

RVV     venin de la vipère de Russel (***Vipera russeli***), ce venin active le facteur V en Va et le facteur X en Xa

RVV-V  fraction purifiée de RVV activant le facteur V en Va

RVV-X  fraction purifiée de RVV activant le facteur X en Xa

VdfP    plasma déficient en facteur V

VdfR    réactif déficient en facteur V

VKA    antagoniste de la vitamine K

### Brève description du dessin

Dans le dessin annexé :

- la figure 1 montre la variation du rapport normalisé n-APC-R-RS, qui est défini plus loin et représenté en ordonnée, en fonction de la concentration (% v/v) du plasma (pool témoin ou plasma APC-R) dans un mélange plasma + VdfP, cette concentration étant représentée en abscisse ; et,
- la figure 2 représente la variation dudit rapport normalisé n-APC-R-RS (en ordonnée) en fonction de la concentration (% v/v) d'un plasma APC-R dans un mélange plasma APC-R + pool témoin de plasmas (en abscisse).

### Description détaillée de l'invention

L'utilisation du venin particulier, CVH, selon l'invention permet d'apprécier la réactivité de APC, et en particulier l'activité fonctionnelle de PC, d'une part, et APC-R, d'autre part. Bien entendu, la détermination de APC-R est plus importante sur le plan du diagnostic que celle de l'activité fonctionnelle de PC. Par suite, la détermination de l'activité fonctionnelle de PC n'est que subsidiaire par rapport à la détermination de APC-R qui permet d'apprécier le risque de thrombose chez le patient testé.

CVH est un produit commercial disponible sur le marché. On peut l'obtenir sous forme purifiée et lyophilisée au près de la société dite SIGMA. Avant l'invention, ce venin était utilisé principalement pour la production d'anti-sérum vis-à-vis des morsures de ***Crotalus viridis helleri.*** En pratique, CVH lyophilisé sera avant emploi dilué avec PB jusqu'à une concentration de 0,10 à 0,15 µg/ml (de préférence à une concentration de 0,12 µg/ml, ce qui correspond lors de la mise en oeuvre du procédé à une concentration finale de 0,03 µg/ml).

Le réactif VdfR est principalement un plasma VdfP. Par l'expression "déficient en facteur V" on entend ici un réactif (VdfR) ou un plasma (VdfP) qui est soit essentiellement appauvri en facteur V soit dépourvu de facteur V. VdfR ou VdfP intervient selon l'invention en tant que plasma substrat, qui peut être d'origine synthétique, animale ou humaine. De façon avantageuse, on fera appel à un VdfP dépourvu de facteur V et obtenu par immunodéplétion. Pour éviter toute interférence avec les anti-phospholipides, notamment ceux du lupus, on recommande d'enrichir VdfR ou VdfP en PL.

Selon un mode particulier de réalisation, à l'étape (1°) on fait appel à un échantillon de plasma humain à tester obtenu par traitement avec du citrate trisodique (lors du prélèvement), centrifugation à 3500 g pendant 15 minutes environ et dilution au moyen de OKB. Le plasma normal utilisé comme témoin à l'étape (4°) est obtenu dans les mêmes conditions.

De façon pratique, en tenant compte des notations APC-R-RS et n-APC-R-RS proposées dans l'article précité de H. DE RONDE et al., à l'étape (3°) et à l'étape (4°) on évalue pour ledit échantillon de plasma humain à tester, d'une part, et pour ledit plasma témoin, d'autre part, la sensibilité à APC, ladite sensibilité étant le rapport (dénommé APC-R-SR) du temps de coagulation en présence de APC au temps de coagulation en l'absence de APC :

$$APC\text{-}R\text{-}SR = \frac{\text{temps de coagulation en présence de APC}}{\text{temps de coagulation en l'absence de APC}}$$

et en ce qu'à l'étape (5°) on apprécie APC-R par le rapport normalisé (dénommé n-APC-R-SR) de la sensibilité à APC de l'échantillon de plasma humain à tester à la sensibilité à APC du plasma normal témoin :

$$n\text{-}APC\text{-}R\text{-}SR = \frac{(APC\text{-}R\text{-}SR)_{\text{échantillon}}}{(APC\text{-}R\text{-}SR)_{\text{plasma témoin}}}$$

Selon le procédé de l'invention, une valeur dudit rapport normalisé n-APC-R-SR > 0,80 indique que l'échantillon de plasma humain à tester ne présente généralement aucune résistance à la protéine C activée, et une valeur dudit rapport normalisé n-APC-R-SR < 0,80 indique que l'échantillon de plasma à tester présente généralement une résistance à la protéine C activée. Le plasma normal utilisé comme témoin est avantageusement un pool de plasmas normaux humains.

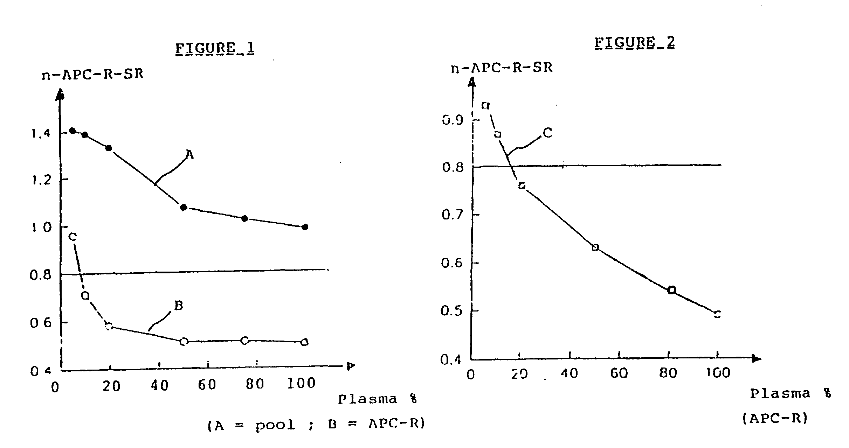

Quand on a une valeur du rapport normalisé comprise dans l'intervalle de doute $(0,72 \leq$ n-APC-R-RS $\leq 0,90)$ pour une concentration x (% v/v) d'un échantillon de plasma dans un mélange de x (plasma) + y (VdfP) où x + y = 100 %, l'on recommande d'augmenter la concentration du plasma, c'est-à-dire inversement de diminuer la concentration de VdfP dans ledit mélange x +y. On obtient ainsi une valeur n-APC-R-RS qui se situe à l'extérieur de l'intervalle de doute (voir figure 1 ci-après).

Avantageusement, on utilisera selon l'invention des volumes identiques pour chacun des réactifs utilisés, à savoir : le plasma humain à tester (de préférence à une dilution de 1/10 dans OKB), VdfR (ou VdfP), solution de CaCl$_2$, solution de CaCl$_2$ + APC, plasma témoin (ou pool de plasmas témoins), et solution (ou dilution) de CVH (de préférence CVH dans de l'eau contenant 1 % v/v de PB).

L'évaluation de APC-R aux étapes (3°), (4°) et (5°) est d'un type connu de l'art antérieur, qui correspond à un taux de conversion d'un "substrat". Le temps de coagulation est mesuré soit directement (notamment au moyen d'un coagulomètre) en repérant l'instant du début de la coagulation, le substrat étant alors VdfP, soit indirectement (notamment au moyen d'un spectromètre) en repérant l'instant correspondant à la formation de thrombine générée dans le système réactionnel, le substrat étant alors la prothrombine. Le substrat préféré selon l'invention est VdfP.

Le nécessaire, kit ou trousse de dosage selon l'invention, comportera de façon avantageuse un VdfP enrichi en PL. Ledit nécessaire peut encore contenir CaCl$_2$ pour préparer la solution aqueuse de CaCl$_2$ à la concentration requise pour fournir les ions Ca$^{2+}$ induisant la coagulation. Le chronomètre est déclenché lors de l'introduction desdits ions Ca$^{2+}$.

Selon le mode préféré de mise en oeuvre du procédé de l'invention :

- à l'étape (1°), on met en contact

  (A) ledit échantillon de plasma humain à tester dilué, notamment au moyen de OKB, à une concentration supérieure ou égale à 1/20 v/v, de préférence à une concentration de 1/10 v/v,
  (B) un plasma humain déficient en facteur V et enrichi en PL, en tant que réactif VdfR, et
  (C) ledit venin de **_Crotalus viridis helleri,_**

  et incube pendant 4 minutes à 37°C ;
- à l'étape (2°), on introduit dans le mélange ainsi incubé (i) Ca$^{2+}$ ou, respectivement, (ii) le mélange Ca$^{2+}$ + APC exogène ;
- à l'étape (3°), on évalue la sensibilité à APC dudit échantillon de plasma humain à tester ;
- à l'étape (4°), on reproduit l'ensemble des étapes (1°) à (3°) où à l'étape (1°) ledit échantillon de plasma humain à tester est remplacé par un plasma humain normal, de façon à évaluer la sensibilité à APC dudit plasma humain normal ; et,
- à l'étape (5°), on apprécie APC-R par le rapport normalisé n-APC-R-SR, une valeur dudit rapport normalisé n-APC-R-SR > 0,80 (de préférence n-APC-R-SR > 0,90) indiquant que l'échantillon de plasma humain à tester ne présente aucune résistance à la protéine C activée, et une valeur dudit rapport normalisé n-APC-R-SR < 0,80 (de préférence n-APC-R-SR < 0,72) indiquant que l'échantillon de plasma humain à tester présente une résistance à la protéine C activée, et le plasma normal utilisé comme témoin étant avantageusement un pool de plasmas normaux humains.

Comme démontré ci-après, le procédé selon l'invention est spécifique et efficace. Il fournit des coefficients de variation "intra-essais" inférieurs à 3 % et des coefficients de variation "inter-essais" inférieurs à 5 %. De plus, il est insensible aux taux de facteurs de la voie intrinsèque, à l'héparine normale (poids moléculaire moyen : 12000 daltons), aux héparines fractionnées de faible poids moléculaire, aux taux de PS, aux taux de facteur VIII, aux CAC, aux VKA et à la mutation 20210A de la prothrombine.

La variante de ce procédé, que l'on recommande eu égard à sa simplicité de mise en oeuvre, son moindre coût et son aptitude à être plus aisément automatisée sur les automates actuels, constitue un procédé amélioré.

Selon cette variante l'on préconise donc un procédé amélioré de détermination de APC-R d'un échantillon de plasma humain à tester, ledit procédé, qui met en oeuvre l'initiation de la coagulation au niveau du facteur X au moyen d'un venin de serpent en présence d'un mélange de Ca$^{2+}$ et APC exogène, puis la mesure du temps de coagulation (T), comprenant les étapes consistant à

(1a) mettre en contact

  (a) un échantillon de plasma humain à tester,
  (b) un réactif déficient en facteur V (VdfR) en tant que produit fournissant la majorité des (de préférence tous les) facteurs de la coagulation autres que le facteur V, et

(c) CVH en tant que produit activant spécifiquement X en Xa, et incuber le mélange ainsi obtenu pendant au moins 1 minute à une température comprise entre 10 et 45°C, de préférence pendant 3 à 5 minutes à une température de 35 à 40°C, et mieux pendant 4 minutes à 37°C ;

(2a) introduire dans ledit mélange ainsi incubé $Ca^{2+}$ et APC exogène ;

(3a) mesurer le temps de coagulation (T) ; et,

(4a) apprécier APC-R par comparaison du temps de coagulation (T) ainsi mesuré avec un temps de coagulation de référence (To) déterminé expérimentalement et ayant statistiquement une valeur d'environ 110 s, T < To indiquant que l'échantillon de plasma humain à tester présente une résistance à la protéine C acavée, et

T > To indiquant que l'échantillon de plasma humain à tester ne présente aucune résistance à la protéine C activée.

Les modalités opératoires données ci-dessus pour les étapes (1°), (2°, ii) et (3°, ii) s'appliquent bien sûr aux étapes (1a), (2a) et (3a) de la variante du procédé de l'invention.

D'un point de vue pratique, To est une plage de 20 s (110 ± 10 s). Ainsi quand T est supérieur à 120 s le plasma testé est normal, et quand T est inférieur à 100 s le plasma testé est à coup sûr APC-R.

Il existe effectivement un intervalle de doute [(en anglais : "grey zone") qui correspond sensiblement à l'intervalle $0,72 \leq$ n-APC-R-RS $\leq 0,90$ précité du procédé principal] quand 100 s $\leq$ T $\leq$ 120 s. Si le temps de coagulation mesuré se situe dans ledit intervalle de doute (100 s $\leq$ T < 120 s), il est recommandé (i) de mettre en oeuvre le procédé principal en augmentant la concentration du plasma (comme indiqué ci-dessus), ou (ii) de réaliser une étude MB qui donnera un résultat non équivoque.

La valeur To = 110 s et surtout l'intervalle de doute 100 s $\leq$ T $\leq$ 120 s ont été déterminés expérimentalement sur plusieurs lots de plasmas humains connus [plasmas normaux (MB-) et plasmas APC-R (MB+)] par mise en contact à 37°C pendant 4 minutes de 1 volume de chaque plasma à étudier (préalablement dilué à 1/10 dans OKB), 1 volume de VdfP enrichi en PL et 1 volume de CVH, introduction dans le mélange résultant de 1 volume d'une solution de APC contenant $CaCl_2$. puis mesure de T. Pour plus de détails voir les exemples 13 et 14 ci-après.

D'autres avantages et caractéristiques de l'invention seront mieux compris à la lecture qui va suivre d'exemples de réalisation et d'essais. L'ensemble de ces éléments n'est pas limitatif mais est fourni à titre d'illustration. Pour information, il est précisé que les exemples 1-3 concernent l'obtention des divers produits et réactifs intervenant selon l'invention, les exemples 4-12 ont trait à la mise en oeuvre du procédé selon l'invention et les exemples 13-14 se rapportent à la variante de ce procédé.

### *Exemple 1*

Obtention du plasma humain à tester

Le prélèvement est effectué sur une solution de citrate trisodique 0,109 M, à raison de 1 volume de citrate pour 9 volumes de sang. L'échantillon est ensuite centrifugé à 3500 g pendant 15 minutes à 4°C. Il est recommandé de faire une double centrifugation à 4°C afin d'éliminer la présence éventuelle de plaquettes dans l'échantillon. La séparation et le recueil du plasma doivent être réalisés le plus rapidement possible après le prélèvement.

Après décantation, le plasma peut être conservé à :

2-8°C pendant 8 h,
15-19°C pendant 8 h, et
-20°C pendant 1 mois.

En cas de décongélation, le plasma doit être décongelé à 37°C au bain-marie thermostaté pendant au moins 10 minutes puis homogénéisé avant utilisation. La recongélation d'un plasma est proscrite.

### *Exemple 2*

Obtention du plasma témoin

On procède comme indiqué à l'exemple 1.

*Exemple 3*

Préparation et conservation des réactifs

- premier réactif (R1)

R1 est un plasma humain lyophilisé immunodéplété en facteur V et enrichi en PL. Le produit lyophilisé est transformé en composition aqueuse au moyen de 2 ml d'eau distillée. On homogénéise la solution résultante à la température ambiante (18-25°C) avant utilisation.

Cette solution est stable à :

18-25°C pendant 6 h,
15-19°C dans l'automate vendu sous la nomenclature commerciale de
"STA" par la société DIAGNOSTICA STAGO pendant 38 h,
2-8°C pendant 26 h, et
-20°C pendant 1 mois.

- deuxième réactif (R2)

R2 est le lyophilisat de CVH. Ce produit lyophilisé est transformé en composition aqueuse au moyen de 2 ml d'eau distillée (pouvant contenir avantageusement 1 % v/v de PB). On homogénéise la composition résultante à la température ambiante (18-25°C) avant utilisation.

Cette composition est stable à :

18-25°C pendant 6 h,
15-19°C dans l'automate "STA" pendant 38 h,
2-8°C pendant 26 h, et
-20°C pendant 1 mois.

- troisième réactif (R3)

R3 est la protéine C activée humaine en milieu calcique lyophilisée. Le lyophilisat est versé dans 1 ml d'eau distillée. On homogénéise la solution résultante à la température ambiante (18-25°C) avant utilisation.

Cette solution est stable à :

18-25°C pendant 6 h,
15-19°C dans l'automate "STA" pendant 38 h,
2-8°C pendant 26 h, et
-20°C pendant 1 mois.

*Exemple 4*

Mode opératoire

On utilise (sauf indication contraire) des volumes identiques des divers produits et réactifs (obtenus selon les exemples 1-3 ci-dessus) qui interviennent dans le procédé de détermination selon l'invention. Ces volumes sont compris entre 5 µl et 50 µl. Les modalités opératoires qui suivent sont celles qui concernent les mesures effectuées au moyen de l'automate "STA" précité ; on a ainsi utilisé :

50 µl d'échantillon de plasma humain à tester (ou de pool de plasmas normaux) dilué à 1/10 dans OKB,
50 µl de R1 (i.e. VdfP enrichi en PL),
50 µl de R2 (i.e. CHV), et
50 µl de $CaCl_2$ en solution aqueuse ou de R3 (i.e. APC + $Ca^{2+}$).

On met en contact au moyen de l'automate "STA" le plasma humain à tester ou le pool de plasmas normaux, VdfP enrichi en PL, et CVH. On incube le mélange résultant pendant 4 minutes à 37°C.

On introduit dans ledit mélange la solution de $CaCl_2$ ou le mélange $CaCl_2$ + APC (i.e. R3) après avoir été chacun préchauffé à 37°C. Le "STA" détermine le temps de coagulation pour l'échantillon à tester et le pool témoin en présence et en l'absence de APC.

Les résultats obtenus sont directement exprimés par l'automate sous la forme du rapport normalisé précité : n-APC-R-RS.

## Exemple 5

### Détermination de la zone normale

A partir de 30 poches de plasmas fournies par le Centre de Transfusion Sanguine, on a déterminé (trois déterminations par essais) la valeur normale du rapport normalisé en fonction d'un pool normal de 25 hommes et de 25 femmes, ainsi que la zone pathologique sur un lot de 15 plasmas MB+ (plasmas APC-R homozygotes et hétérozygotes). On a pu constater que la valeur normale pour les plasmas MB- est telle que n-APC-R-RS ≥ 0,80 avec l'automate "STA".

## Exemple 6

### Reproductibilité intra-essais

A partir de 2 plasmas APC-R homozygotes (MB+), notés APC-R 1 et APC-R 2, et d'un pool normal intervenant comme témoin (MB-), 21 déterminations ont été effectuées en triple. Les résultats concernant le coefficient de variation sont donnés dans le tableau I qui suit. Ils montrent que le coefficient de variation du rapport normalisé n-APC-R-RS est inférieur à 3 %.

## TABLEAU I

| | Coefficient de variation de n-APC-R-RS | | |
|---|---|---|---|
| | Lot 005 | Lot 006 | Lot 007 |
| Pool MB- | 2,08 | 1,94 | 2,25 |
| APC-R 1 (MB+) | 1,64 | 0,96 | 1,32 |
| APC-R 2 (MB+) | 1,60 | 1,82 | 1,57 |

## Exemple 7

### Reproductibilité jour-par-jour

Afin d'apprécier la reproduction jour-par-jour, un plasma normal congelé (MB-) et 2 plasmas pathologiques (MB+) l'un congelé et l'autre lyophilisé ont été aliquotés puis testés pendant 10 jours avec le même lot de réactifs sur le même automate "STA". Les résultats obtenus sont consignés dans le tableau II. Ils montrent que le coefficient de variation de n-APC-R-RS jour-par-jour est inférieur à 5 %.

## TABLEAU II

|  | MB- | MB+ congelé | MB+ lyophilisé |
|---|---|---|---|
|  | n-APC-R-RS | n-APC-R-RS | n-APC-R-RS |
| M | 0,93 | 0,51 | 0,56 |
| SD | 0,03 | 0,02 | 0,02 |
| CV % | 3,66 | 3,99 | 4,08 |

Notes :

M : moyenne de 10 essais par plasma

SD : écart standard par rapport à la moyenne

CV % : coefficient de variation

### Exemple 8

Reproductibilité par lot

On a testé avec l'automate "STA" un plasma normal MB- et un plasma pathologique MB+ (plasma APC-R hétérozygote) aliquotés et congelés. Aussitôt après la décongélation, chaque plasma a été testé cinq fois dans une même série (un flacon), 15 séries indépendantes (15 flacons du même lot) ont été réalisées. Les résultats consignés dans le tableau III montrent que le coefficient de variation est inférieur à 5 %.

TABLEAU III

| T ou rapport | Plasma normal (MB-) | | | Plasma pathologique (MB+) | | |
|---|---|---|---|---|---|---|
|  | M | SD | CV % | M | SD | CV % |
| T sans APC | 41,93 s | 0,757 s | 1.804 | 38,46 s | 0,602 s | 1,564 |
| T avec APC | 138,86 s | 5,898 s | 4,247 | 59,42 s | 1,568 s | 2,639 |
| APC-R-RS | 3,31 | 0,157 | 4,738 | 1,54 | 0.035 | 2,236 |
| n-APC-R-RS (médiane) | 1,230 | 0,052 | 4,242 | 0,575 | 0,010 | 1.807 |

Notes :

T : temps de coagulation (en secondes)

M : moyenne des essais

SD : écart standard par rapport à la moyenne

CV : coefficient de variation (exprimé en %)

### Exemple 9

Reproductibilité lot par lot

2 plasmas pathologiques notés MB+ 1 et MB+2 (plasmas homozygotes) et un plasma normal MB- ont été décongelés et étudiés avec 3 lots différents des mêmes réactifs (moyenne de 5 mesures par essai). Un second plasma normal identique au précédent a été également testé en parallèle afin de déterminer le rapport n-APC-R-RS. Les résultats consignés dans le tableau IV montrent que le procédé selon l'invention est reproductible lot par lot.

TABLEAU IV

| Plasmas testés | n-APC-R-RS | | | |
|---|---|---|---|---|
| | Lot 005 | Lot 006 | Lot 007 | Δ max |
| MB- | 1,17 | 1,10 | 1,07 | 0,10 |
| MB+ 1 | 0,54 | 0,52 | 0,55 | 0,03 |
| MB+ 2 | 0,55 | 0.52 | 0,54 | 0,03 |
| Note : | | | | |
| Δ max : variation maximale | | | | |

Selon le tableau IV, Δ max (en valeur absolue) est inférieur ou égal à 0,05 pour les plasmas MB+ et inférieur ou égal à 0,10 pour le plasma MB-.

### Exemple 10

Influence du taux de facteur V

Pour mettre en évidence la spécificité du procédé selon l'invention en ce qui concerne le facteur V, on a réalisé une série de dilutions d'un pool normal ou d'un plasma APC-R dans un VdfP, d'une part, et une série de dilutions du même plasma APC-R dans ledit pool normal, d'autre part.

Les résultats obtenus ont été consignés dans les figures 1 et 2.

La courbe A de la figure 1 donne la variation du rapport normalisé n-APC-R-SR en fonction de la concentration (en % v/v) du pool de plasmas normaux dans le mélange (pool de plasmas normaux) + (VdfP).

La courbe B de ladite figure 1 donne la variation du rapport normalisé n-APC-R-SR en fonction de la concentration (en % v/v) du plasma APC-R dans le mélange (plasma APC-R) + (VdfP).

La courbe C de la figure 2 donne la variation du rapport normalisé n-APC-R-SR en fonction de la concentration (en % v/v) du même plasma APC-R dans le mélange (plasma APC-R) + (pool de plasmas normaux).

Les courbes A et B mettent en évidence que :

- en ce qui concerne la dilution du plasma APC-R dans un VdfP, on obtient un résultat "positif" (n-APC-R-SR $\leq 0,71$) quand le plasma APC-R est à une concentration de 10 à 100 % v/v ; et,
- en ce qui concerne la dilution du pool de plasmas normaux dans un VdfP, on obtient un résultat "négatif" (n-APC-R-SR $\geq 0,98$) quand ledit pool de plasmas normaux est à une concentration de 10 à 100 % v/v.

En conséquence, les courbes A et B de la figure 1 démontrent clairement la spécificité du procédé selon l'invention en ce qui concerne la mutation de Leiden du facteur V.

Cette spécificité est confirmée par la courbe C de la figure 2 qui montre que la dilution du plasma APC-R dans le pool de plasmas normaux conduit à un résultat "positif" (n-APC-R-SR < 0,80) dès la concentration de 20 % v/v de plasma APC-R dans le mélange (APC-R) + (pool de plasmas normaux).

### Exemple 11

Essai comparatif 1

Pour comparaison on a testé sur automate "STA" 180 plasmas, à savoir 60 MB- et 120 MB+ (60 homozygotes et 60 hétérozygotes, notés respectivement MB+ A et MB+ B) en utilisant comme initiateur de la coagulation soit RVV-X selon l'enseignement de EP-A-0711838 [avec un rapport volumique VdfP/(VdfP + plasma à tester) = 75 %] soit CVH selon l'invention [avec un rapport volumique VdfP/(VdfP + plasma à tester) = 50 %]. Les résultats consignés dans le tableau V montrent que le procédé selon l'invention fournit une meilleure sensibilité. Dans cet essai comparatif, un second pool de 50 plasmas normaux MB- a été utilisé comme témoin.

TABLEAU V

| Nombre de plasmas testés | | | | | | |
|---|---|---|---|---|---|---|
| Plasmas utilisés | contrôle par MB | | selon l'art antérieur | | selon l'invention | |
| | + | - | + | - | + | - |
| MB- | 0 | 60 | 0 | 60 | 0 | 60 |
| MB+ A | 60 | 0 | 59 | 1 | 60 | 0 |
| MB+ B | 60 | 0 | 57 | 3 | 60 | 0 |

*Exemple 12*

Essai comparatif 2

En s'inspirant de l'enseignement de WO-A-9604560 [à savoir ici un seul venin (RVV-X) pour l'activation X→Xa], on a comparé RVV-X avec CVH dans la détermination de APC-R. A cet effet, on a testé sur l'automate "STA" précité deux plasmas pathologiques (notés APC-R 1 et respectivement APC-R 2) dilués à 1/10 dans OKB vis-à-vis d'un pool de plasmas normaux, en utilisant en outre deux contrôles : un plasma MB- et un plasma MB+.Les résultats obtenus consignés dans le tableau VI ci-après, mettent en évidence que CVH présente un pouvoir discriminant supérieur à celui de RVV-X. En effet, pour les plasmas APC-R 1 et APC-R 2, RVV-X donne un rapport normalisé n-APC-R-SR se situant dans l'intervalle de doute $0,72 \leq$ n-APC-R-SR $\leq 0,90$ (0,72 pour APC-R1 ; 0,77 pour APC-R 2), alors que CVH donne un rapport normalisé (0,57 pour APC-R 1 ; 0,58 pour APC-R 2) nettement inférieur au seuil minimal (0,72) dudit intervalle de doute.

## TABLEAU VI

| | Plasmas | | | | |
|---|---|---|---|---|---|
| | Pool normal | MB- contrôle | MB+ contrôle | APC-R 1 | APC-R 2 |
| **RVV-X** | | | | | |
| T (s) | 36,6/79,9 | 42,3/86,8 | 40,2/52,4 | 43,4/63,2 | 45,0/70,6 |
| ΔT (s) | 40,3 | 44,5 | 12,2 | 19,8 | 25,6 |
| rs | 2,01 | 2,05 | 1,30 | 1,45 | 1,56 |
| rn | - | 1,02 | 0,64 | 0,72 | 0,77 |
| **CVH** | | | | | |
| T (s) | 39,3/114,4 | 43,8/118,0 | 42,1/71,1 | 43,0/71,9 | 46,0/78,0 |
| ΔT (s) | 75,1 | 74,2 | 29,0 | 28,9 | 32,0 |
| rs | 2,91 | 2,69 | 1,68 | 1,67 | 1,69 |
| rn | - | 0,92 | 0,57 | 0,57 | 0,58 |
| Notes : | | | | | |
| T : temps de coagulation (en secondes) sans APC/avec APC | | | | | |
| ΔT : variation du temps de coagulation T(avec APC) - T(sans APC) | | | | | |
| rs : APC-R-SR | | | | | |
| rn : n-APC-R-SR | | | | | |

### Exemple 13

Mode opératoire

On utilise des volumes identiques de plasma à étudier et de réactifs R1, R2 et R3 obtenus respectivement selon les exemples 1 et 3 ci-dessus. Ces volumes sont compris entre 5 µl et 50 µl. Les modalités opératoires qui suivent sont celles qui concernent les mesures effectuées au moyen de l'automate "STA" précité ; on a ainsi utilisé :

50 µl d'échantillon de plasma humain à tester dilué à 1/10 dans OKB,
50 µl de R1 (i.e. VdfP enrichi en PL),
50 µl de R2 (i.e. CHV), et
50 µl de R3 (i.e. APC + Ca$^{2+}$).

On met en contact au moyen de l'automate "STA" le plasma humain à tester, VdfP enrichi en PL, et CVH. On incube le mélange résultant pendant 4 minutes à 37°C.
On introduit dans le mélange ainsi obtenu R3 (i.e. CaCl$_2$ + APC) après l'avoir préchauffé à 37°C. Le "STA" détermine le temps de coagulation (T) pour l'échantillon de plasma à tester.
En fonction du résultat obtenu trois cas se présentent :

- si T > 120 s. le plasma testé n'est pas APC-R [il s'agit d'un plasma normal (MB-)],
- si 120 s $\leq$ T $\leq$ 100 s, il y a un doute (pour lever ce doute, on opère comme indiqué plus haut : soit selon le procédé principal, soit par MB), et
- si T < 100 s, le plasma testé est un plasma APC-R (plasma MB+).

Le tableau III ci-dessus illustre un tel résultat, le temps de coagulation (avec APC) du plasma MB- étant de 138,86 s, et celui du plasma MB+ de 59,42 s. Il en est de même pour le tableau VI en ce qui concerne les essais mettant en oeuvre CVH.

### Exemple 14

Efficacité

En procédant selon les modalités de l'exemple 13, on a étudié 582 plasmas humains répartis en deux lots et analysés chacun par MB, le lot 1 comprenant 398 plasmas (14 plasmas MB+ et 384 plasma MB-) et le lot 2 comprenant 184 plasmas (93 plasmas MB+ et 91 plasmas MB-). Les résultats obtenus sont consignés dans le tableau VII ci-après, où chaque temps de coagulation (T) est apprécié par périodes de 10 s (de 60 à 160 s) et au-delà de 160 s.

TABLEAU VII

| Temps de coagulation T (en seconde) | Plasmas testés | |
|---|---|---|
| | Lot 1 (n = 398) (A) | Lot 2 (n = 184) (B) |
| 60<T<70 | 5 MB+ | 5 MB+ |
| 70<T$\leq$80 | 8 MB+ | 38 MB+ |
| 80<T$\leq$90 | 1 MB+ | 37 MB+ |
| 90<T<100 | 0 | 12 MB+ |
| 100$\leq$T$\leq$110 | 0 | 1 MB+ |
| 110<T$\leq$120 | 2 MB- | 0 |
| 120<T$\leq$130 | 5 MB- | 0 |
| 130<T$\leq$140 | 42 MB- | 2 MB- |
| 140<T$\leq$150 | 82 MB- | 5 MB- |

TABLEAU VII   (suite)

| Temps de coagulation T (en seconde) | Plasmas testés | |
|---|---|---|
| | Lot 1 (n = 398) (A) | Lot 2 (n = 184) (B) |
| 150 < T≤160 | 86 MB- | 7 MB- |
| T > 160 | 167 MB- | 77 MB- |
| **Notes** | | |
| A : Nombre et nature des plasmas du lot 1 ayant coagulé | | |
| B : Nombre et nature des plasmas du lot 2 ayant coagulé | | |

Les résultats du tableau VII mettent en évidence que la technique de la détermination utilisée (i) ne donne aucun faux positif ni aucun faux négatif dans les lots 1 et 2 (i.e. aucun plasma MB- se retrouve classé parmi les plasmas MB + et réciproquement aucun plasma MB + se retrouve classé parmi les plasmas MB- ), et (ii) ne fournit qu'un nombre restreint (< 0,6%) de valeurs douteuses [parmi les 582 plasmas testés, seuls 3 plasmas se situent dans l'intervalle de doute (100 s < T ≤ 120 s), à savoir : 2 plasmas MB- pour le lot 1 et 1 plasma MB+ pour le lot 2].

**Revendications**

**1.** Utilisation d'un venin de serpent pour déclencher la coagulation par activation du facteur X en Xa en vue de déterminer la réactivité de la protéine C activée, notamment l'activité fonctionnelle de la protéine C, d'un plasma humain à tester, ladite utilisation étant **caractérisée en ce que** l'on fait appel à du venin de *Crotalus viridis helleri* en tant que substance initiant la coagulation, en présence de $Ca^{2+}$, au niveau du facteur X, ledit venin activant sélectivement X en Xa sans être influencé par la présence ou l'absence des autres facteurs de la coagulation.

**2.** Utilisation suivant la revendication 1, **caractérisée en ce que** l'on fait appel à du venin de *Crotalus viridis helleri* pour déterminer la résistance (APC-R) à la protéine C activée (APC) d'un plasma humain à tester.

**3.** Procédé de détermination de APC-R d'un échantillon de plasma humain à tester, ledit procédé, qui met en oeuvre l'initiation de la coagulation au niveau du facteur X au moyen d'un venin de serpent en présence (i) de $Ca^{2+}$ ou (ii) d'un mélange de $Ca^{2+}$ et APC exogène, puis l'évaluation du temps de coagulation, (i) en l'absence de APC et, respectivement, (ii) en présence de APC, par rapport à un plasma normal, comprenant les étapes consistant à

(1°) mettre en contact

(a) un échantillon de plasma humain à tester,
(b) un réactif déficient en facteur V (VdfR) en tant que produit fournissant la majorité des facteurs de la coagulation autres que le facteur V, et
(c) le venin de *Crotalus viridis helleri* en tant que produit activant spécifiquement X en Xa,

et incuber le mélange ainsi obtenu pendant au moins 1 minute à une température comprise entre 10 et 45°C;
(2°) introduire dans ledit mélange ainsi incubé (i) $Ca^{2+}$ ou, respectivement, (ii) $Ca^{2+}$ + APC exogène ;
(3°) évaluer le temps de coagulation (i) en l'absence de APC et, respectivement, (ii) en présence de APC ;
(4°) reproduire les étapes (1°) à (3°) en remplaçant à l'étape (1°) ledit échantillon de plasma humain à tester par un plasma normal en tant que témoin ; et,
(5°) apprécier APC-R par la comparaison des évaluations obtenues aux étapes (3°) et (4°).

**4.** Procédé suivant la revendication 3, **caractérisé en ce que** ledit réactif VdfR est un plasma humain ou animal déficient en facteur V (VdfP).

**5.** Procédé suivant la revendication 3, **caractérisé en ce que** l'on introduit à l'étape (1°) des phospholipides (PL).

**6.** Procédé suivant la revendication 3, **caractérisé en ce que**, à l'étape (1°) et à l'étape (4°) on fait appel à un échantillon de plasma humain à tester obtenu par traitement avec du citrate trisodique, centrifugation à 3500 g pendant 15 minutes et dilution au moyen du tampon d'Owren-Koller (OKB), d'une part, et à un plasma témoin

EP 0 956 509 B1

obtenu à partir de sang humain normal et dilué dans des conditions identiques à celle dudit échantillon de plasma humain à tester, d'autre part.

7. Procédé suivant la revendication 3, **caractérisé en ce que**, à l'étape (3°) et à l'étape (4°) on évalue pour ledit échantillon de plasma humain à tester, d'une part, et pour ledit plasma témoin, d'autre part, la sensibilité à APC, ladite sensibilité étant le rapport (dénommé APC-R-SR) du temps de coagulation en présence de APC au temps de coagulation en l'absence de APC :

$$APC\text{-}R\text{-}SR = \frac{\text{temps de coagulation en présence de APC}}{\text{temps de coagulation en l'absence de APC}}$$

et en ce qu'à l'étape (5°) on apprécie APC-R par le rapport normalisé (dénommé n-APC-R-SR) de la sensibilité à APC de l'échantillon de plasma humain à tester à la sensibilité à APC du plasma normal témoin :

$$n\text{-}APC\text{-}R\text{-}SR = \frac{(APC\text{-}R\text{-}SR)_{échantillon}}{(APC\text{-}R\text{-}SR)_{plasma\ témoin}}$$

le plasma normal utilisé comme témoin étant avantageusement un pool de plasmas normaux humains.

8. Procédé suivant la revendication 3, **caractérisé en ce que** :

- à l'étape (1°), on met en contact

    (A) ledit échantillon de plasma humain à tester dilué, notamment au moyen de OKB, à une concentration supérieure ou égale à 1/20 v/v,
    (B) un plasma humain déficient en facteur V (VdfP) et enrichi en PL, en tant que réactif VdfR, et
    (C) ledit venin de *Crotalus viridis helleri,*

    et incube pendant 4 minutes à 37°C ;
- à l'étape (2°), on introduit dans le mélange ainsi incubé (i) Ca$^{2+}$ ou, respectivement, (ii) le mélange Ca$^{2+}$ + APC exogène ;
- à l'étape (3°), on évalue la sensibilité à APC dudit échantillon de plasma humain à tester ;
- à l'étape (4°), on reproduit l'ensemble des étapes (1°) à (3°) où à l'étape (1°) ledit échantillon de plasma humain à tester est remplacé par un plasma humain normal, de façon à évaluer la sensibilité à APC dudit plasma humain normal ; et,
- à l'étape (5°), on apprécie APC-R par le rapport normalisé n-APC-R-SR, une valeur dudit rapport normalisé n-APC-R-SR > 0,80 indiquant généralement que l'échantillon de plasma humain à tester ne présente aucune résistance à la protéine C activée, et une valeur dudit rapport normalisé n-APC-R-SR < 0,80 indiquant généralement que l'échantillon de plasma humain à tester présente une résistance à la protéine C activée, et le plasma normal utilisé comme témoin étant avantageusement un pool de plasmas normaux humains.

9. Procédé suivant la revendication 3, **caractérisé en ce qu'**il comprend la mise en oeuvre des étapes (1°), (2°, ii) et (3°, ii), puis l'appréciation de APC-R par comparaison du temps de coagulation (T) obtenu à l'étape (3, ii) avec un temps de coagulation de référence (To) déterminé expérimentalement,

    T < To indiquant généralement que l'échantillon de plasma humain à tester présente une résistance à la protéine C activée, et
    T > To indiquant généralement que l'échantillon de plasma humain à tester ne présente aucune résistance à la protéine C activée.

10. Procédé suivant la revendication 9, **caractérisé en ce que** To a statistiquement une valeur d'environ 110 s.

11. Procédé suivant la revendication 9, pour la détermination de APC-R d'un échantillon de plasma humain à tester, ledit procédé, qui met en oeuvre l'initiation de la coagulation au niveau du facteur X au moyen d'un venin de serpent en présence d'un mélange de Ca$^{2+}$ et APC exogène, puis la mesure du temps de coagulation (T) comprenant les étapes consistant à

(1a) mettre en contact

(a) un échantillon de plasma humain à tester,
(b) un réactif déficient en facteur V (VdfR) en tant que produit fournissant la majorité des (de préférence tous les) facteurs de la coagulation autres que le facteur V, et
(c) le venin de *Crotalus viridis helleri* en tant que produit activant spécifiquement X en Xa, et incuber le mélange ainsi obtenu pendant au moins 1 minute à une température comprise entre 10 et 45°C, de préférence pendant 3 à 5 minutes à une température de 35 à 40°C, et mieux pendant 4 minutes à 37°C ;

(2a) introduire dans ledit mélange ainsi incubé $Ca^{2+}$ et APC exogène ;
(3a) mesurer le temps de coagulation (T) ; et,
(4a) apprécier APC-R par comparaison du temps de coagulation (T) ainsi mesuré avec un temps de coagulation de référence (To) déterminé expérimentalement et ayant Statistiquement une valeur d'environ 110 s, T < To indiquant que l'échantillon de plasma humain à tester présente une résistance à la protéine C activée, et T > To indiquant que l'échantillon de plasma humain à tester ne présente aucune résistance à la protéine C activée.

**12.** Procédé suivant la revendication 9 ou 11, **caractérisé en ce que** To est une plage de 20 s (110 ± 10 s) telle que :

- • quand T > 120 s, le plasma testé ne présente aucune résistance à la protéine C activée, et,
- • quand T < 100 s, le plasma testé présente une résistance à la protéine C activée,
- • l'intervalle de doute étant 100 s ≤ T ≤ 120 s.

**13.** Nécessaire de dosage utile pour mettre en oeuvre le procédé de l'une quelconque des revendications 3 à 12, **caractérisé en ce qu'**il comprend :

- - un premier réactif qui est un VdfP,
- - un second réactif constitué par le venin de *Crotalus viridis helleri*, **et**
- - un troisième réactif constitué par APC en milieu calcique.

**14.** Nécessaire de dosage selon la revendication 13, **caractérisé en ce que** ledit premier réactif est enrichi en PL.


**Claims**

**1.** A use of a snake venom to initiate coagulation by activating factor X into Xa in order to determine the reactivity of activated protein C (APC), in particular the functional activity of protein C, of a human plasma to be tested, said use being **characterized in that** the venom of *Crotalus viridis helleri* is used as the substance initiating coagulation, in the presence of $Ca^{2+}$, at the level of factor X, said venom activating selectively X into Xa without being influenced by the presence or absence of the other coagulation factors.

**2.** A use according to claim 1, **characterized in that** the venom of *Crotalus viridis helleri* is used to determine the resistance (APC-R) to activated protein C (APC) of a human plasma to be tested.

**3.** A method for determining the APC-R of a human plasma sample to be tested, said method which uses the initiation of clotting at the level factor X by means of a snake venom in the presence (i) of $Ca^{2+}$ or (ii) of a mixture of $Ca^{2+}$ and exogenic APC, then evaluating the clotting time, (i) in the absence of APC and, respectively, (ii) in the presence of APC, in comparison to a normal plasma, comprising steps consisting of

(1°) bringing into contact

(a) a human plasma sample to be tested,
(b) a factor V deficient reagent (VdfR), as a product supplying most of the coagulation factors other than factor V, and
(c) the venom of *Crotalus viridis helleri* as a product specifically activating X into Xa,

and incubating the mixture thus obtained for at least 1 minute at a temperature of from 10°C to 45°C ;
(2°) introducing into said mixture thus incubated (i) $Ca^{2+}$ or, respectively, (ii) $Ca^{2+}$ + exogenic APC ;

(3°) evaluating the clotting time (i) in the absence of APC and, respectively, (ii) in the presence of APC ;

(4°) repeating steps (1°) to (3°) by replacing in step (1°) said human plasma sample to be tested with a normal plasma as control; and

(5°) estimating APC-R by comparing the evaluations obtained in steps (3°) and (4°).

4. A method according to claim 3, **characterized in that** said VdfR reagent is a human or animal factor V deficient plasma (VdfP).

5. A method according to claim 3, **characterized in that** phospholipids (PL) are introduced in step (1°).

6. A method according to claim 3, **characterized in that**, in step (1°) and step (4°) use is made of a human plasma sample to be tested, which is obtained by treatment with trisodium citrate, centrifugation at 3500 g for 15 minutes, and dilution with Owen-Koller buffer (OKB) on the one hand, and of a control plasma, which is obtained from normal human blood and diluted under conditions identical to those of said human plasma sample on the other hand.

7. A method according to claim 3, **characterized in that**, in step (3°) and in step (4°) the sensitivity to APC of said human plasma sample and of said control plasma is evaluated, said sensitivity being the ratio (called APC-R-SR) of the clotting time in the presence of APC to the clotting time in the absence of APC :

$$\text{APC-R-SR} = \frac{\text{coagulation time in presence of APC}}{\text{coagulation time in absence of APC}}$$

and in that, in step (5°) APC-R is reckoned by the standardized ratio (called n-APC-R-SR) of the sensitivity to APC of the human plasma sample to be tested, to the sensitivity to APC of the normal control plasma :

$$\text{n-APC-R-SR} = \frac{\text{(APC-R-SR) sample}}{\text{(APC-R-SR) control plasma}}$$

the normal plasma used as control being advantageously a pool of normal human plasmas.

8. A method according to claim 3, **characterized in that**,

- in step (1°), the following are brought into contact:

   (A) said human plasma sample to be tested diluted, in particular with OKB, to a concentration higher than or equal to 1/20 v/v,
   (B) a human factor V deficient plasma (VdfP) and enriched with PL, as VdfR reagent, and
   (C) said venom of *Crotalus viridis helleri*, and incubated for 4 minutes at 37°C ;

- in step (2°), (i) $Ca^{2+}$ or, respectively, (ii) the mixture $Ca^{2+}$ + exogenic APC, is introduced into the mixture thus incubated ;
- in step (3°) the sensitivity to APC of said human plasma sample to be tested is evaluated;
- in step (4°), all of steps (1°) to (3°) wherein said human plasma sample to be tested is replaced with a normal human plasma are repeated so as to evaluate the sensitivity to APC of said normal human plasma ; and
- in step (5°), APC-R is evaluated by the standardized ratio n-APC-R-SR, a value of said standardized ratio n-APC-R-SR > 0.80 generally indicating that the human plasma sample to be tested has no resistance to the activated protein C, and a value of said standardized ratio n-APC-R-SR < 0.80 generally indicating that the human plasma sample to be tested does have resistance to the activated protein C, and the normal plasma used as control is advantageously a pool of normal human plasmas.

9. A method according to claim 3, **characterized in that** it comprises carrying out steps (1°) (2°, ii) and (3°, ii), then determining APC-R by comparison of the clotting time (T) obtained in step (3°, ii) with a reference clotting time ($T_o$) determined by experiment,

T < $T_o$ generally indicating that the human plasma sample to be tested is resistant to APC, and

T > $T_o$ generally indicating that the human plasma sample to be tested is not resistant to APC.

10. A method according to claim 9, **characterized in that** $T_o$ statistically has a value of about 110 seconds.

11. A method according to claim 9, for the determination of APC-R of a human plasma sample to be tested, said method, which uses the initiation of clotting at the factor X level by means of a snake venom in the presence of a mixture of $Ca^{2+}$ and exogenic APC, then measuring the clotting time (T), comprising the steps consisting of:

(1a) bringing into contact

(a) a human plasma sample to be tested,
(b) a factor V deficient reagent (VdfR) as a product providing most (preferably all) of the clotting factors other than factor V, and
(c) the venom of *Crotalus viridis helleri* as a product specifically activating X to Xa,

and incubating the mixture thus obtained for one minute at a temperature of from 10°C to 45°C, preferably for 3 to 5 minutes at a temperature of from 35°C to 40°C, and more preferably for 4 minutes at 37°C ;
(2a) introducing $Ca^{2+}$ and exogenic APC into said mixture thus incubated;
(3a) measuring the clotting time (T) ; and
(4a) evaluating APC-R by comparing the clotting time (T) thus measured with an experimentally determined reference clotting time ($T_o$) having statistically a value of about 110 seconds,

$T < T_o$ indicating that the human plasma sample to be tested is resistant to APC, and
$T > T_o$ indicating that the human plasma sample to be tested is not resistant to APC.

12. A method according to claim 9 or 11, **characterized in that** $T_o$ is a range of 20 s (110 ± 10 s), such that:

- when T > 120 s, the tested human plasma sample is not resistant to APC, and
- when T < 100 s, the tested human plasma sample is resistant to APC,
- the interval of doubt being 100 s $\leq$ T < 120 s.

13. A dosage kit useful for carrying out the method of any one of claims 3 to 12, **characterized in that** it includes :

- a first reagent which is a VdfP,
- a second reagent consisting of the venom of *Crotalus viridis helleri*, and
- a third reagent consisting of APC in a calcium-containing medium.

14. A dosage kit according to claim 13, **characterized in that** the said first reagent is enriched with PL.


**Patentansprüche**

1. Verwendung von Schlangengift zur Auslösung der Koagulation eines zu testenden menschlichen Plasmas durch Aktivierung des Faktors X zu Xa zum Bestimmen der Reaktivität von aktiviertem Protein C, insbesondere der funktionellen Aktivität von Protein C, wobei die Verwendung **dadurch gekennzeichnet ist, daß** *Crotalus viridis helleri*-Gift in Gegenwart von $Ca^{2+}$ auf der Stufe des Faktors X als die Koagulation initiierende Substanz verwendet wird, wobei das Gift selektiv X zu Xa aktiviert, unabhängig von der Gegenwart oder Abwesenheit anderer Koagulationsfaktoren.

2. Verwendung nach Anspruch 1, **dadurch gekennzeichnet, daß** *Crotalus viridis helleri*-Gift zum Bestimmen der Resistenz (APC-R) eines zu testenden menschlichen Plasmas gegenüber aktiviertem Protein C (APC) verwendet wird.

3. Verfahren zum Bestimmen der APC-R einer zu testenden menschlichen Plasmaprobe, wobei das Verfahren die Koagulation auf der Stufe des Faktors X mittels eines Schlangengifts in der Gegenwart (i) von $Ca^{2+}$ oder (ii) einem Gemisch aus $Ca^{2+}$ und exogenem APC initiiert, und anschließend das Bestimmen der Koagulationszeit in Bezug auf normales Plasma (i) in Abwesenheit von APC bzw. (ii) in Gegenwart von APC, umfassend die Schritte bestehend aus

(1.) Inkontaktbringen

(a) einer zu testenden menschlichen Plasmaprobe,

(b) eines Reagens mit Faktor V-Mangel (VdfR) als Produkt, das die Mehrzahl der Koagulationsfaktoren außer dem Faktor V zur Verfügung stellt, und

(c) *Crotalus viridis helleri*-Gift als spezifisch X zu Xa aktivierendes Produkt, und Inkubieren des erhaltenen Gemisches für mindestens 1 Minute bei einer Temperatur zwischen 10° und 45°C;

(2.) Einbringen von (i) $Ca^{2+}$ oder, bzw., (ii) $Ca^{2+}$ + exogenem APC in das inkubierte Gemisch;

(3.) Bestimmen der Koagulationszeit (i) in Abwesenheit von APC bzw. (ii) in Gegenwart von APC;

(4.) Wiederholen der Schritte (1.) bis (3.), wobei in Schritt (1.) die zu testende menschliche Plasmaprobe durch normales Plasma als Nachweis ersetzt wird; und

(5.) Bestimmen der APC-R durch Vergleich der in den Schritten (3.) und (4.) erhaltenen Werte.

4. Verfahren nach Anspruch 3, **dadurch gekennzeichnet, daß** das Reagens VdfR ein menschliches oder tierisches Plasma mit Faktor V-Mangel (VdfP) ist.

5. Verfahren nach Anspruch 3, **dadurch gekennzeichnet, daß** in Schritt (1.) Phospholipide (PL) eingebracht werden.

6. Verfahren nach Anspruch 3, **dadurch gekennzeichnet, daß** in Schritt (1.) und Schritt (4.) einerseits eine Probe von zu testendem menschlichen Plasma verwendet wird, erhalten durch Behandlung mit Trinatriumcitrat, Zentrifugation bei 3500 g für 15 Minuten und Lösung mittels Owren-Koller-Puffer (OKB), andererseits ein Nachweisplasma, erhalten ausgehend von normalem menschlichen Blut und gelöst unter denselben Bedingungen wie die der zu testenden menschlichen Plasmaprobe, verwendet wird.

7. Verfahren nach Anspruch 3, **dadurch gekennzeichnet, daß** in Schritt (3.) und Schritt (4.) für die zu testende menschliche Plasmaprobe einerseits und für das Nachweisplasma andererseits die Sensibilität gegenüber APC bestimmt wird, wobei die Sensibilität das Verhältnis (genannt APC-R-SR) zwischen der Koagulationszeit in Gegenwart von APC und der Koagulationszeit in Abwesenheit von APC ist:

$$APC\text{-}R\text{-}SR = \frac{\text{Koagulationszeit in Gegenwart von APC}}{\text{Koagulationszeit in Abwesenheit von APC}}$$

und dadurch, daß in Schritt (5.) der APC-R bestimmt wird durch das normalisierte Verhältnis (genannt n-APC-R-SR) zwischen der Sensibilität der zu testenden menschlichen Plasmaprobe gegenüber APC und der Sensibilität gegenüber dem normalen Nachweisplasma:

$$n\text{-}APC\text{-}R\text{-}SR = \frac{(APC\text{-}R\text{-}SR) \; Muster}{(APC\text{-}R\text{-}SR) \; Nachweisplasma}$$

wobei das als Nachweis verwendete normale Plasma vorzugsweise ein Pool normalen menschlichen Plasmas ist.

8. Verfahren nach Anspruch 3, **dadurch gekennzeichnet, daß**:

- in Schritt (1.)

   (A) die gelöste, zu testende menschliche Plasmaprobe, insbesondere mittels OKB, in einer Konzentration von größer oder gleich 1/20 Volumen-%,

   (B) menschliches mit PL angereichertes Plasma mit Faktor V-Mangel (VdfP) als Reagens VdfR und

   (C) *Crotalus viridis helleri*-Gift

   in Kontakt gebracht und für 4 Minuten bei 37°C inkubiert werden;

- in Schritt (2.) (i) $Ca^{2+}$ oder, bzw., (ii) das $Ca^{2+}$ + exogenes APC-Gemisch in das inkubierte Gemisch eingebracht werden;

- in Schritt (3.) die Sensibilität der zu testenden menschlichen Plasmaprobe gegenüber APC bestimmt wird;

- in Schritt (4.) die Gesamtheit der Schritte (1.) bis (3.) wiederholt werden, wobei in Schritt (1.) die zu testende menschliche Plasmaprobe durch normales menschliches Plasma zum Bestimmen der Sensibilität des normalen menschlichen Plasmas gegenüber APC ersetzt wird; und

- in Schritt (5.) der APC-R durch das normalisierte Verhältnis n-APC-R-SR bestimmt wird, wobei ein Wert des normalisierten Verhältnisses n-APC-R-SR von > 0,80 im allgemeinen anzeigt, daß die zu testende menschliche Plasmaprobe keinen Widerstand gegenüber aktiviertem Protein C aufweist, und ein Wert des normalisierten Verhältnisses n-APC-R-SR von < 0,80 im allgemeinen anzeigt, daß die zu testende menschliche Plasmaprobe einen Widerstand gegenüber aktiviertem Protein C aufweist, und wobei das als Nachweis verwendete normale Plasma vorzugsweise ein Pool normalen menschlichen Plasmas ist.

9.   Verfahren nach Anspruch 3, **dadurch gekennzeichnet, daß** es das Umsetzen der Schritte (1.), (2., ii) und (3., ii) und nachfolgend das Bestimmen der APC-R durch Vergleich der in Schritt (3., ii) erhaltenen Koagulationszeit (T) mit einer experimentell bestimmten Referenzkoagulationszeit (To) umfaßt,

wobei T < To im allgemeinen anzeigt, daß die zu testende menschliche Plasmaprobe einen Widerstand gegenüber aktiviertem Protein C aufweist, und
T > To im allgemeinen anzeigt, daß die zu testende menschliche Plasmaprobe keinen Widerstand gegenüber aktiviertem Protein C aufweist.

10.   Verfahren nach Anspruch 9, **dadurch gekennzeichnet, daß** To einen statistischen Wert von ungefähr 110 s hat.

11.   Verfahren nach Anspruch 9 zum Bestimmen der APC-R einer zu testenden menschlichen Plasmaprobe, wobei das Verfahren die Initiierung der Koagulation mittels eines Schlangengifts auf der Stufe des Faktors X in Gegenwart eines Gemisches aus $Ca^{2+}$ und exogenem APC und nachfolgend das Messen der Koagulationszeit (T) umfaßt, umfassend die Schritte bestehend aus

(1a) Inkontaktbringen

(a) einer zu testenden menschlichen Plasmaprobe,
(b) eines Reagens mit Faktor V-Mangel (VdfR) als Produkt, das die Mehrzahl der (vorzugsweise alle) Koagulationsfaktoren außer dem Faktor V zur Verfügung stellt, und
(c) *Crotalus viridis helleri*-Gift als spezifisch X zu Xa aktivierendes Produkt,

und Inkubieren des erhaltenen Gemisches für mindestens 1 Minute bei einer Temperatur zwischen 10° und 45°C, vorzugsweise zwischen 3 und 5 Minuten bei einer Temperatur von 35° bis 40°C, und mehr bevorzugt für 4 Minuten bei 37°C;
(2a) Einbringen von $Ca^{2+}$ + exogenem APC in das inkubierte Gemisch;
(3a) Messen der Koagulationszeit (T); und
(4a) Bestimmen der APC-R durch Vergleich der gemessenen Koagulationszeit (T) mit einer experimentell bestimmten Referenzkoagulationszeit (To) mit einem statistischen Wert von ungefähr 110 s,

wobei T < To anzeigt, daß die zu testende menschliche Plasmaprobe einen Widerstand gegenüber aktiviertem Protein C aufweist, und
T > To anzeigt, daß die zu testende menschliche Plasmaprobe keinen Widerstand gegenüber aktiviertem Protein C aufweist.

12.   Verfahren nach Anspruch 9 oder 11, **dadurch gekennzeichnet, daß** To eine Zeitspanne von 20 s ist (110± 10 s), wobei:

•   wenn T > 120 s, das getestete Plasma keinen Widerstand gegenüber aktiviertem Protein C aufweist, und
•   wenn T < 100 s, das getestete Plasma einen Widerstand gegenüber aktiviertem Protein C aufweist,
•   wobei die Zeitspanne von 100s≤T≤120s unbestimmt ist.

13.   Kit zur Verwendung bei der Umsetzung des Verfahrens nach einem der Ansprüche 3 bis 12, **dadurch gekennzeichnet, daß** es umfaßt:

-   ein erstes Reagens, das ein VdfP ist,
-   ein zweites Reagens, das *Crotalus viridis helleri*-Gift ist, und
-   ein drittes Reagens, das APC in calciumhaltigem Medium ist.

14.   Kit nach Anspruch 13, **dadurch gekennzeichnet, daß** das Reagens mit PL angereichert ist.

FIGURE_1

FIGURE_2